(19) Europäisches Patentamt
European Patent Office
Office européen des brevets

(11) Publication number: **0 001 149**
**B1**

(12) # EUROPEAN PATENT SPECIFICATION

(45) Date of publication of patent specification: **03.06.81**

(51) Int. Cl.³: **C 07 D 501/24,**
**C 07 D 501/00**

(21) Application number: **78200174.7**

(22) Date of filing: **06.09.78**

(54) **Process for the preparation of 3-bromomethyl-3-cephem-sulphoxide derivatives.**

(30) Priority: **07.09.77 GB 3741377**

(43) Date of publication of application:
**21.03.79 Bulletin 79/6**

(45) Publication of the grant of the European patent:
**03.06.81 Bulletin 81/22**

(84) Designated Contracting States:
**BE CH DE FR GB LU NL SE**

(56) References cited:
**GB - A - 1 326 531**

(73) Proprietor: **Gist - Brocades N.V.**
**Wateringseweg 1 P.O. Box 1**
**NL-2600 MA Delft (NL)**

(72) Inventor: **Bruynes, Cornelis Adrianus**
**Ridderhoflaan 51**
**NL-2396 CK Koudekerk a/d Rijn (NL)**
Inventor: **De Koning, Jan Jacobus**
**Burg. Elsenlaan 80**
**NL-2283 AC Rijswijk-ZH (NL)**

(74) Representative: **Mars, Pieter et al,**
**c/o Gist-Brocades N.V. Patents and Trademarks**
**P.O.Box 1**
**NL-2600 MA Delft (NL)**

**EP 0 001 149 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Process for the preparation of 3-bromomethyl-3-cephem-sulphoxide derivatives

This invention relates to an improved process for the preparation of 3-bromomethyl-3-cephem sulphoxide derivatives.

3-Bromomethyl-3-cephem sulphoxide derivatives of the general formula:

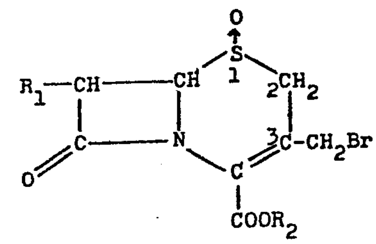

I

wherein $R_1$ represents an acylamino group and $R_2$ represents a group protecting the carboxy radical, are valuable intermediates for the preparation of various therapeutically useful cephalosporanic acid derivatives. The bromine atom can readily be replaced by various nucleophilic atoms or groups as is disclosed in, for example British Patent Specification 1326531 to give 3'-nucleophilic substituted desacetoxy-cephalosporanic acid derivatives which are, or can be converted by methods known *per se* into, antibiotically active cephalosporanic derivatives.

The acylamido group $R_1$ can be any group—hitherto disclosed in the chemical literature (including patent specifications) or kown to those skilled in the art of penicillin or cephalosporin chemistry—attached to the 6-position in natural or synthetic penicillin compounds or attached to the 7-position of natural or synthetic cephalosporin compounds. Preferably the acylamino group is one present in the 6$\beta$-side chain of penicillins that can be obtained by fermentative procedures, which penicillins can readily be converted into cephem sulphoxides by known methods. Suitable groups represented by $R_1$ are, for example, phenylacetamido, phenoxyacetamido, benzamido and formamido.

The group $R_2$ can be any group known to those skilled in the art for protecting the carboxy group of penicillanic acid or cephalosporanic acid derivatives, but preferably $R_2$ represents an ester group which can be readily introduced and easily removed again after the reaction has been completed. Suitable ester groups are, for example, straight- or branched-chain alkyl groups having from 1 to 4 carbon atoms optionally substituted by one or more halogen atoms or by one or two phenyl groups, which phenyl groups may themselves carry one or more substituents such as nitro and methoxy groups, and silyl groups, for example trialkylsilyl groups. Particularly suitable ester groups are the methyl, t-butyl, 2-bromoethyl, 2,2,2-trichloroethyl, benzyl, 4-nitrobenzyl, 4-methoxybenzyl, benzhydryl and trimethylsilyl groups.

Hitherto, known compounds of general formula I have been prepared by bromination in manner known per se of corresponding desacetoxy-3-cephem sulphoxides of the general formula:

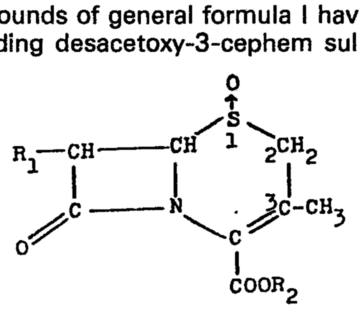

II

(wherein $R_1$ and $R_2$ are as hereinbefore defined), and separation of the desired 3-bromomethyl-3-cephem sulphoxides of general formula I from the reaction mixture; see for example, British Patent Specification 1326531. This straightforward procedure does not give very satisfactory yields of the desired products, *inter alia* because side-reactions can take place whereby bromine is introduced in other positions than that of the 3-methyl group of the compounds of general formula II, for example in the 2-position; see again the British Patent cited above, page 5, lines 4 to 8.

It has been found that the yields of the 3-bromomethyl-3-cephem sulphoxides of general formula I can be substantially improved if the reaction mixture obtained by bromination of the sulphoxides of general formula II, which contain *inter alia* 2-bromo-3-bromomethyl-3-cephem sulphoxides of the general formula:

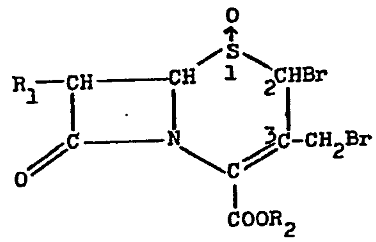

III

(wherein $R_1$ and $R_2$ are as hereinbefore defined) is treated with a debrominating agent so as to replace the bromine atom in the 2-position by a hydrogen atom, thus converting an undesired side-reaction product of the bromination reaction also into the desired 3-bromomethyl-3-cephem sulphoxide derivative of general formula I.

According to the present invention, there is provided an improved process for the preparation of 3-bromo-methyl-3-cephem sulphoxide derivatives of general formula I by brominating in manner known *per se* a desacetoxy-3-cephem sulphoxide of general formula II to convert *inter alia* the methyl group in the 3-position of such a compound to a bromomethyl group characterized by treating the reaction mixture that is obtained after the bromination has been effected, containing a compound of general formula III, or optionally a compound of general formula III separated from such a reaction mixture, with a debrominating agent selected from the group consisting of trivalent organic phosphorus compounds and inorganic salts which are capable of replacing—in the presence of a hydrogen donor—the bromine atom in the 2-position by hydrogen, and recovering by methods known *per se* the 3-bromomethyl-3-cephem sulphoxide derivative of general formula I thus produced.

Another undesired side-reaction product which can be present in the reaction mixture obtained after bromination of the compounds of general formula II, is the corresponding 2-bromo-3-methyl-3-cephem sulphoxide. When this product is present in said reaction mixture it will be converted back again to the starting material of general formula II during the debromination reaction described above.

This conversion is an additional advantage of the process of the invention since it prevents loss of starting material in the form of the undesired 2-bromo-3-methyl derivative, as it is converted into the starting material which can then be used again in the improved bromination process of the invention.

By the process of the present invention the yields of the 3-bromomethyl-3-cephem sulphoxide derivatives of general formula I obtained can be improved considerably as compared with the known process for the bromination of desacetoxy-3-cephem sulphoxides of the general formula II.

Bromination of a desacetoxy-3-cephem sulphoxide of general formula II to convert *inter alia* the methyl group in the 3-position to a bromomethyl group can be effected by means of known brominating agents, such as N-bromoimides or N-bromoamides. Suitable brominating agents are, for example, 1,3-dibromo-5,5-dimethylhydantoin and N-bromosuccinimide.

The necessary activation of the brominating agent can be achieved by adding a free radical initiator to the reaction mixture or by irradiation of the reaction mixture with ultraviolet or visible light, e.g. electric light from a 150—500 W lamp. Suitable free radical initiators are, for example, azo compounds, such as azo-isobutyronitrile, and peroxides such as benzoyl peroxide.

The reaction is preferably carried out in a suitable inert organic solvent, for example, methylene chloride or 1,2-dichloroethane.

When a free radical initiator is used for the activation of the brominating agent, the reaction is advantageously carried out at a temperature between 40° and 90°C; in the case when the activation is effected by irradiation of the reaction mixture with light, the temperature of the reaction mixture is preferably between −20° and 30°C.

There is usually obtained a mixture of bromination products, consisting of the 2-bromo-3-methyl, the 2-bromo-3-bromomethyl and the 3-bromomethyl derivatives of the starting 3-cephem sulphoxides of general formula II.

The quantities of the various bromination products thus obtained can be established by means of High Performance Liquid Chromatography (HPLC) analysis and depend on the nature of the starting sulphoxide of general formula II, the brominating agent and bromination technique actually used and, generally, on the other reaction conditions employed during the bromination process.

The various bromination products can be isolated from the reaction mixture in manner known *per se*, for example, the reaction mixture may be evaporated to dryness and the products may be separated by chromatography of the residue on silica gel.

As aforementioned, the quantities of the various products in the reaction mixture can be established by means of HPCL analysis. In this method the bromination products are first isolated in the manner described hereinbefore. Then the content of the products and that of the starting material is determined by means of quantitative NMR analysis. These compounds are then used as references in the HPLC analysis in order to determine the quantities of the various products in the reaction mixture, before and after the debromination has been carried out.

Before this analysis method is effected, the reaction mixtures have to be diluted in order to obtain

concentrations suitable for this method. The values given in the Examples hereinafter are always in relation to such diluted mixtures. This method of analysis has an accuracy of approximately 10%.

After the bromination of the desacetoxy-3-cephem sulphoxides of general formula II, the reaction mixture containing 2-bromo-3-methyl, 2-bromo-3-bromomethyl- and 3-bromomethyl-3-cephem sulphoxides is treated with a debrominating agent in the presence of a hydrogen donor to convert the 2-bromo-3-bromomethyl compound of general formula III present therein to the desired 3-bromomethyl compound of general formula I, and concurrently also a 2-bromo-3-methyl-3-cephem sulphoxide back again to a starting material of general formula II, which can then be used again.

Accordingly, after the bromination and debromination reactions have been carried out, there is usually obtained a mixture mainly consisting of a 3-bromomethyl-3-cephem sulphoxide of general formula I in admixture with some of the corresponding starting material of general formula II.

The 3-bromomethyl-3-cephem sulphoxide of formula I thus obtained can be isolated from the reaction mixture in manner known *per se*, for example, as described above for the mixture of bromination products.

Alternatively, a 2-bromo-3-bromomethyl-3-cephem sulphoxide of general formula III present in the reaction mixture can, after separation and optional purification, by itself be treated with a debrominating agent in the presence of a hydrogen donor to convert it to a 3-bromomethyl compound of the formula I.

The debromination is preferably carried out in a suitable organic solvent, for example, acetone, acetonitrile, acetic acid, dioxan, ethyl acetate, methylene chloride, 1,2-dichloroethane, 1,2-dimethoxyethane, tetrahydrofuran, 2-methoxyethanol, propylene carbonate, N,N-dimethylacetamide, bis(2-methoxyethyl)ether or tetrahydrothiophene1,1-dioxide.

Debromination of a 2-bromo-3-bromomethyl-3-cephem sulphoxide of general formula III—as such or as present in the reaction mixture in which it is formed by the bromination of a compound of general formula II—to a 3-bromomethyl-3-cephem sulphoxide of general formula I is effected by treatment—in the presence of a hydrogen donor—with a trivalent organic phosphorus compound or an inorganic salt as the debrominating agent. Suitable debrominating agents are selected from the group consisting of

(a) phosphites of the general formula:

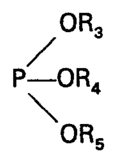

IV

wherein $R_3$, $R_4$ and $R_5$ are the same or different and each represents a straight- or branched-chain alkyl group which may be substituted by a chlorine atom and has from 1 to 18 carbon atoms, a phenyl group (which may be substituted by a methyl group or a chlorine atom), an allyl or benzyl group;

(b) other phosphites, for example phenyl neopentylglycol phosphite, poly(dipropyleneglycol) phenyl phosphite, tris 25 phosphite and tris(dipropyleneglycol) phosphite;

(c) di- and triphosphites, for example diisodecyl pentaerythritol diphosphite, tetraphenyl dipropyleneglycol diphosphite, distearyl pentaerythritol diphosphite, diphenyl didecyl (2,2,4-trimethyl-1,3-pentanediol) diphosphite, tetrakis (nonylphenyl) polypropyleneglycol diphosphite and heptakis (dipropyleneglycol) triphosphite;

(d) phosphonites, for example di(lower alkyl) phenylphosphonites;

(e) phosphinites, for example lower alkyl diphenylphosphinites;

(f) phosphines, for example chlorodiphenylphosphine;

(g) other trivalent phosphorus compounds, for example hexamethylphosphorous triamide, 2-ethoxy-4-methyl-1,3,2-dioxaphospholane, 4-methyl-2-piperidino-1,3,2-dioxaphospholane, diethyl N-methyl-N-phenylphosphoramidite and bis-o-phenylene pyrophosphite;

(h) inorganic salts, for example stannous chloride ($SnCl_2$), sodium sulphite ($Na_2SO_3$) and sodium dithionite ($Na_2S_2O_4$).

By the term "lower alkyl" as employed herein, is meant straight- or branched-chain alkyl groups containing from 1 to 4 carbon atoms, such as methyl, ethyl and t-butyl.

The debromination step is preferably carried out with at least an equimolar quantity of the debrominating agent in relation to the 2-bromo-3-bromomethyl compound of general formula III and at ambient or somewhat lower temperature. When the debrominating agent is of group (h) just mentioned above, the ratio between the reactants should be approximately equimolar; when the debromination agent is of groups (a) to (g) however, a rather substantial excess of debrominating agent can be used as well.

Suitable debrominating agents of formula IV which may be used in the process of the invention are, for example, trimethyl phosphite, triethyl phosphite, tripropyl phosphite, triisopropyl phosphite, tributyl phosphite, triisobutyl phosphite, triamyl phosphite, trioctyl phosphite, triisooctyl phosphite, tris(2-ethylhexyl) phosphite, tridecyl phosphite, triisodecyl phosphite, trineodecyl

phosphite, trilauryl phosphite, tristearyl phosphite, triallyl phosphite, tris(2-chloroethyl) phosphite, triphenyl phosphite, tris(p-methylphenyl) phosphite, tris-o-tolyl phosphite, tris(p-chlorophenyl) phosphite, 2-ethylhexyl dioctyl phosphite, diethyl benzyl phosphite, didecyl phenyl phosphite, diisodecyl phenyl phosphite, isooctyl diphenyl phosphite, 2-ethylhexyl diphenyl phosphite, decyl diphenyl phosphite and isodecyl diphenyl phosphite.

Suitable phosphonites which may be used in the process of the invention are, for example, dimethyl phenylphosphonite, diethyl phenylphosphonite and di(t-butyl) phenylphosphonite.

Suitable phosphinites are, for example, methyl diphenylphosphinite and ethyl diphenylphosphinite.

Particularly suitable are the following debrominating agents: trimethyl phosphite, triethyl phosphite, tributyl phosphite, triisooctyl phosphite, triisodecyl phosphite, trilauryl phosphite, tristearyl phosphite, triallyl phosphite, tris(2-chloroethyl) phosphite, triphenyl phosphite, tris-o-tolyl phosphite, tris(p-chlorophenyl) phosphite, 2-ethylhexyl dioctyl phosphite, diethyl benzyl phosphite, isooctyl diphenyl phosphite, 2-ethylhexyl diphenyl phosphite, isodecyl diphenyl phosphite, phenyl neopentylglycol phosphite, poly(dipropyleneglycol) phenyl phosphite, tris 25 phosphite, tris(dipropyleneglycol) phosphite, diisocedyl pentaerythritol diphosphite, tetraphenyl dipropyleneglycol diphosphite, distearyl pentaerythritol diphosphite, diphenyl didecyl (2,2,4-trimethyl-1,3-pentanediol) diphosphite, tetrakis(nonylphenyl) polypropyleneglycol diphosphite, heptakis (dipropyleneglycol) triphosphite, bis-o-phenylene pyrophosphite, hexamethylphosphorous triamide, 2-ethoxy-4-methyl-1,3,2-dioxaphospholane, 4-methyl-2-piperidino-1,3,2-dioxaphospholane, diethyl N-methyl-N-phenylphosphoramidite, diethyl phenylphosphonite, methyl diphenylphosphinite and chlorodiphenylphosphine.

The debromination of *inter alia* the 2-bromo-3-bromomethyl 3-cephem sulphoxides of general formula III should be carried out in the presence of a hydrogen donor; such a donor is usually already present in the reaction medium, for example in the form of water, etc. But in some cases it may be advantageous to add a hydrogen donor such as water, a lower alkanol, e.g. methanol, or a carboxylic acid, e.g. acetic acid, to the reaction medium.

The starting materials of general formula II which are brominated are known products or can be prepared by methods known *per se.* By the term "methods known *per se"* as used in this specification is meant methods heretofore used or described in the chemical literature.

The following Examples illustrate the process of the invention. To distinguish the various types of compounds in the reaction mixtures, the following notation is used throughout the Examples:

I : starting material
II : corresponding 3-bromomethyl derivative
III : corresponding 2-bromo derivative
IV : corresponding 2-bromo-3-bromomethyl derivative

IR spectra were taken as dispersions in KBr.

Example I

(a) A mixture of 103.2 g of dicyclohexylcarbodiimide, 43.5 g of t-butyl alcohol and 1.1 g of cuprous chloride was kept at room temperature for 3 days. The mixture was then dissolved in 200 ml of methylene chloride and the solution was added dropwise into a suspension of 35 g of 7-benzamido-3-methyl-3-cephem-4-carboxylic acid 1-oxide in 500 ml of methylene chloride. After stirring for 24 hours at room temperature, the precipitated N,N'-dicyclohexyl urea was removed by filtration and the precipitate washed with methylene chloride. The combined filtrates were washed sequentially with 2N hydrochloric acid, saturated $NaHCO_3$ solution (three times) and with water (twice). Methylene chloride was removed by evaporation *in vacuo* and toluene was added to the residue. The crystalline solid was isolated by filtration and washed with toluene. Upon crystallisation from methylene chloride/toluene, there were obtained 19.6 g (48% yield) of t-butyl 7-benzamido-3-methyl-3-cephem-4-carboxylate 1-oxide.

NMR ($CDCl_3$): 1.56 (s, 9H), 2.14 (s, 3H), 3.11 3.41, 3.52, 3.83 (ABq, 2H, J 18.5 Hz), 4.65 (br., 1H), 6.25 (dd, 1H, J 4.5 and 9.5 Hz), 7.37—7.97 (m, 5H), 7.56 (d, 1H, J 9.5 Hz)
IR: 3400, 1770, 1715, 1685, 1520, 1030 $cm^{-1}$.

(b) To a solution of 7.8 g (20 mmoles) of the product of (a) in 150 ml of methylene chloride, under nitrogen and cooled in an ice bath, were sequentially added 4.2 ml (30 mmoles) of triethylamine and 3.28 g (24 mmoles) of N-bromosuccinimide; the mixture was irradiated with a 150 W tungsten lamp for 15 minutes. The dark-brown reaction mixture was washed with a dilute hydrochloric acid solution (pH 1) and then twice with 250 ml portions of water. The organic layer was dried over magnesium sulphate, filtered and evaporated to dryness. There were obtained 10.6 g (purity 62%) of t-butyl 7-benzamido-2-bromo-3-methyl-3-cephem-4-carboxylate 1-oxide; yield 70%.
NMR ($CDCl_3$): 1.56 (s, 9H), 2.21 (s,3H), 5.21 (s, 1H) 5.25 (d, 1H, J 4.8 Hz); 6.33 (dd, 1H, J 4.8 and 10

Hz), 7.3—7.96 (m, 5H), 7.41 (d, 1H, J 10 Hz).
IR: 3400, 1800, 1730, 1675, 1520, 1055 $cm^{-1}$.

(c) 11.7 g (30 mmoles) of the product of (a) were dissolved in 250 ml of methylene chloride. The solution was kept under nitrogen and cooled in ice, and then 5.3 ml (45 mmoles) of triethylamine and 6.4 g (36 mmoles) of N-bromosuccinimide were subsequently added. The resulting solution was irradiated with a 150 W tungsten lamp for 20 minutes. The dark brown solution was washed with dilute hydrochloric acid (pH 1) and with water. After drying over magnesium sulphate, the solution was concentrated by evaporation to approximately 100 ml. To the solution of t-butyl 7-benzamido-2-bromo-3-methyl-3-cephem-4-carboxylate 1-oxide thus obtained were added 100 ml of acetic acid. The solution was kept under nitrogen, cooled in ice and irradiated with a 150 W tungsten lamp. 7.15 g (40 mmoles) of N-bromosuccinimide were added, following after 4 hours by another 1.78 g (10 mmoles) of the same compound. Then the solution was irradiated with a 150 W tungsten lamp for another 3 hours. Parts of the solution thus obtained (52%), which contained t-butyl 7-benzamido-2-bromo-3-bromomethyl-3-cephem-4-carboxylate 1-oxide, was washed 4 times with 100 ml of water. After drying over magnesium sulphate, the solution was concentrated by evaporation to approximately 25 ml. Then 5 ml of methanol were added and the solution was treated with activated charcoal. After addition of 1,2-dichloroethane the solution was evaporated to dryness. The residue was dissolved in diethyl ether, and the product was precipitated with n-heptane. There were obtained 6.8 g (79.4% yield) of t-butyl 7-benzamido-2-bromo-3-bromomethyl-3-cephem-4-carboxylate 1-oxide.

NMR ($CDCl_3$+DMSO—$D_6$): 1.59 (s, 9H), 4,17, 4,34, 4.58, 4.75 (ABq, 2H, J 10.5 Hz), 5.34 (d, 1H, J 5 Hz), 5.73 (s, 2H), 6.35 (dd, 1H, J 5 and 9.5 Hz), approximately 7.34—7.98 (m, 5H), 7.71 (d, 1H, J 9.5 Hz).
IR: 3380, 1790, 1725, 1680, 1520, 1050 $cm^{-1}$.

(d) The remainder of the solution of the 2-bromo-3-bromomethyl compound obtained was used as such in the following debromination process.

To this solution, which contained at most 14.4 mmoles of t-butyl 7-benzamido-2-bromo-3-bromomethyl-3-cephem-4-carboxylate 1-oxide, were added 14 ml (approximately 25 mmoles) of triisodecyl phosphite, and the mixture was stirred for 30 minutes at room temperature.

The reaction mixture was then washed with water, dried over magnesium sulphate and filtered. The filtrate was evaporated to dryness. Upon addition of a small quantity of acetone a brown precipitate was formed; upon addition of diethyl ether the brown precipitate was decoloured almost completely. After standing for one night in the refrigerator, the precipitate was filtered off to give 3.0 g (44.6% yield) of t-butyl 7-benzamido-3-bromomethyl-3-cephem-4-carboxylate 1-oxide.

NMR ($CDCl_3$—DMSO—$D_6$): 1.57 (s, 9H) 3.44, 3.74, 3.79, 4.09 (ABq, 2H, J 18 Hz), 4.26, 4.42, 4.51, 4.67 (ABq, 2H, J 10.5 Hz) 4.90 (d, 1H, J 4.8 Hz), 6.20 (dd, 1H, J 4.8 and 9.5 Hz), 7.35—7.96 (m, 5H), 7.85 (d, 1H, J 9.5 Hz).
IR: 3330, 1795, 1715, 1645, 1520, 1030 $cm^{-1}$.

The compound thus obtained was used as a reference in the HPLC analysis described below.

(e) 248.1 mg of the product of (c) (with a purity of 78% according to quantitative NMR analysis) were dissolved in 10 ml of acetone. To the solution was added 0.05 ml of acetic acid and, after cooling in an ice bath, 0.2 ml of trimethyl phosphite. The mixture was kept in the ice bath for another 10 minutes and then acetone was added until the volume of the mixture was exactly 25 ml. 5 ml of this solution was diluted once more with acetone to 25 ml and 5 ml of the solution thus obtained were used in the HPLC analysis. It was found that the solution contained 1.17 mg of t-butyl 7-benzamido-3-bromomethyl-3-cephem-4-carboxylate 1-oxide per ml; it could be concluded that the yield of the debromination was 89%.

The remaining solutions were united and evaporated to dryness. After drying for one night *in vacuo* at room temperature there were obtained 242.2 mg of residue. If the debromination process had proceeded quantitatively, the residue would contain 66% of t-butyl 7-benzamido-3-bromomethyl-3-cephem-4-carboxylate 1-oxide; the quantitative NMR analysis indicated a 68% content.

Thus, it could be concluded that the debromination process had indeed proceeded quantitatively.

## Example II

(a) To a solution of 2.02 g (5 mmoles) of t-butyl 7-phenylacetamido-3-methyl-3-cephem-4-carboxylate 1-oxide in 50 ml of dry methylene chloride, under a nitrogen atmosphere and cooled in an ice bath, were added over 1 hour portions of 1.71 g (9.6 mmoles ) of N-bromosuccinimide, while the mixture was irradiated with a 150 W tungsten lamp. The irradiation was continued for another 1.5 hours and then the solvent was removed by evaporation. The residue was dissolved in a small quantity of acetone, whereupon the products were precipitated by adding petroleum ether (boiling from 60° to 80°C). There were obtained 2.36 g of solid compounds.

(b) 625 mg of the solids thus obtained were dissolved in 40 ml of 1,2-dichloroethane and after filtering the solution was diluted with acetone to 100 ml; 50 ml of this solution were diluted again with acetone to 25 ml.

According to HPLC (High Performance Liquid Chromatography) analysis the solution contained per ml:

$1.0 \times 10^{-3}$ mmoles of t-butyl 3-bromomethyl-7-phenylacetamido-3-cephem-4-carboxylate 1-oxide (compound II),

$0.17 \times 10^{-3}$ mmoles of t-butyl 2-bromo-3-methyl-7-phenylacetamido-3-cephem-4-carboxylate 1-oxide (compound III),

$0.66 \times 10^{-3}$ mmoles of t-butyl 2-bromo-3-bromomethyl-7-phenylacetamido-3-cephem-4-carboxylate 1-oxide (compound IV).

The starting material in the bromination process, i.e. t-butyl 7-phenylacetamido-3-methyl-3-cephem-4-carboxylate 1-oxide (compound I), could not be detected anymore in the reaction mixture.

To other 5 ml portions of the original solution was each added at room temperature a phosphite as specified below; these solutions were also each diluted with acetone to 25 ml.

The results of the HPLC analysis of these solutions are summarized in the Table below.

| Phosphorus compound | HPLC analysis ($10^{-3}$ mmoles/ml) | |
|---|---|---|
| | I | II |
| trimethyl phosphite | 0.18 | 1.7 |
| triisooctyl phosphite | 0.18 | 1.8 |
| triisodecyl phosphite | 0.18 | 1.7 |
| triphenyl phosphite | 0.17 | 1.8 |

It may be concluded that compound III (2-bromo) and compound IV (2-bromo-3-bromomethyl) were completely converted by the phosphites into the corresponding debrominated products.

(c) The reference compound IV in the above HPLC analysis was prepared as follows:

To a solution of 4.6 g (9.5 mmoles) of t-butyl 2-bromo-7-phenylacetamido-3-methyl-3-cephem-4-carboxylate 1-oxide in 350 ml of methylene chloride, cooled in ice and irradiated with a 150 W tungsten lamp, were added in portions over a period of 105 minutes 3.05 g (17.1 mmoles) of N-bromosuccinimide. After the addition was completed, the irradiation was continued for another hour. The orange-coloured solution was concentrated to dryness by evaporation at low temperature. The residue was chromatographed on silica gel (elution with methylene chloride/acetone; first 72:1, then 49:1, v/v). There was obtained 1.4 g (26% yield) of t-butyl 2-bromo-3-bromomethyl-7-phenylacetamido-3-cephem-4-carboxylate 1-oxide (compound IV).

NMR ($CDCl_3$): 1.55 (s, 9H), 3.58 (s, 2H), 4.16, 4.61 (ABq, 2H, J 11 Hz), 5.10 (d, 1H, J 5 Hz), 5.52 (s, 1H), 6.07 (dd, 1H, J 5 and 10 Hz), 6.76 (d, 1H, J 10 Hz), 7.26 (s, 5H).

IR: 3350, 3260, 1790, 1705, 1670, 1500, 1060 $cm^{-1}$.

## Example III

(a) A solution of 2-bromoethyl 7-phenylacetamido-3-methyl-3-cephem-4-carboxylate 1-oxide was brominated according to the procedure described in Example II(a); the experiment was repeated.

(b) The reaction mixtures obtained were subjected to a series of debromination reactions according to the procedure described in Example II(b) by means of the phosphites listed below. The results of the HPLC analysis of the reaction mixtures resulting from the debromination processes, are summarized in the following Table.

The first line in each Table relates to the HPLC analysis of the reaction mixture before the debromination reaction was carried out.

| Phosphorus compound | HPLC analysis ($10^{-3}$ mmoles/ml) | | |
|---|---|---|---|
| | I | II | III |
| Exp. 1. | | | |
| – | – | 2.3 | 0.08 |
| tributyl phosphite | 0.13 | 2.8 | – |
| triphenyl phosphite | 0.10 | 2.6 | – |
| diphenyl 2-ethylhexyl phosphite | 0.06 | 2.7 | – |
| diphenyl isooctyl phosphite | 0.07 | 2.6 | – |
| triisodecyl phosphite | 0.12 | 2.6 | – |
| Exp. 2. | | | |
| – | 0.32 | 2.6 | 0.28 |
| trimethyl phosphite | 0.46 | 2.7 | – |
| triethyl phosphite | 0.46 | 2.8 | – |
| triallyl phosphite | 0.44 | 2.8 | – |
| triisooctyl phosphite | 0.44 | 2.8 | – |

(c) The reference compound II in this HPLC analysis, i.e. 2-bromoethyl 3-bromomethyl-7-phenylacetamido-3-cephem-4-carboxylate 1-oxide was prepared as follows.

To an ice-cooled solution of 2.28 g (5 mmoles) of 2-bromoethyl 7-phenylacetamido-3-methyl-3-cephem-4-carboxylate 1-oxide in 20 ml of 1,2-dichloroethane and 20 ml of acetic acid, which was kept under nitrogen and which was irradiated with a 150 W tungsten lamp, were added in portions over a period of 2 hours 1.42 g (8 mmoles) of N-bromosuccinimide. The starting material had then disappeared completely. The solvent was removed by evaporation and 150 ml of ethyl acetate were added and the solution obtained was washed sequentially with 2 portions of 250 ml of water, with a sodium bicarbonate solution and finally with water until neutral reaction. After drying over magnesium sulphate and filtering, the solution was evaporated until it was almost completely dry, and then 150 ml of diethyl ether were added and the precipitate was collected by filtration. There was obtained 1.1 g (41% yield) of 2-bromoethyl 3-bromomethyl-7-phenylacetamido-3-cephem-4-carboxylate 1-oxide.

NMR (DMSO—$D_6$): 3.40, 3.72, 3.88, 4.20 (ABq, 2H, J 19 Hz), 3.65 (s, 2H), 3.75 (t, 2H, J 6 Hz), 4.40, 4.56, 4.61, 4.77 (ABq, 2H, J 10 Hz), 4.64 (t, 2H, J 6 Hz), 4.99 (d, 1H, J 4.8 Hz), 5.89 (dd, 1H, J 4.8 and 8.8 Hz), 7.32 (s, 5H), 8.43 (d, 1H, J 8.8 Hz).

IR: 3280, 1790, 1730, 1650, 1525, 1030 $cm^{-1}$.

(d) The reference compound III in the HPLC analysis of the debromination mixture of (b), i.e. 2-bromoethyl 2-bromo-7-phenylacetamido-3-methyl-3-cephem-4-carboxylate 1-oxide, was prepared according to the procedure described in Example I(b); 2.58 g (5.7 mmoles) of 2-bromoethyl 7-phenylacetamido-3-methyl-3-cephem-4-carboxylate 1-oxide were thus converted into 2.50 g (82.5% yield) of the corresponding 2-bromo derivative.

NMR ($CDCl_3$): 2.20 (s, 3H), 3.53 (t, 2H, J 6 Hz), 3.60 (s, 2H), 4.35, 4.55, 4.59 (t ABq, 2H, J 6 and 12 Hz), 5.11 (s, 1H), 5.12 (d, 1H, J 4.8 Hz), 6.11 (dd, 1H, J 4.8 and 9.5 Hz), 6.86 (d, 1H, J 9.5 Hz), 7.29 (s, 5H).

IR: 3270, 1795, 1735, 1680, 1515, 1055 $cm^{-1}$.

(e) The starting material in the procedures of (a), (c) and (d), i.e. 2-bromoethyl 7-phenylacetamido-3-methyl-3-cephem-4-carboxylate 1-oxide, was prepared according to the procedure described in Example I(a); it can be characterized as follows:

NMR (DMSO—$D_6$): 2.05 (s, 3H, 3.38, 3.61, 3.64, 3.88 (ABq, 2H, J 14 Hz), 3.68 (s, 2H), 3.71 (t, 2H, J 5.5 Hz), 4.57 (t, 2H, J 5.5 Hz), 4.87 (d, 1H, 4.5 Hz), 5.77 (dd, 1H, J 4.5 and 8.5 Hz), 7.30 (s, 5H), 8.32 (d, 1H, J 8.5 Hz).

IR: 3280, 1770, 1730, 1655, 1530, 1035 $cm^{-1}$.

The reference compound IV was not available. Accordingly, although a quantitative increase of the concentration of compound II during the debromination process could be established, it was not possible to correlate this with a corresponding quantitative decrease of the concentration of compound IV.

Example IV

(a) A solution of t-butyl 7-formamido-3-methyl-3-cephem-4-carboxylate 1-oxide was brominated according to the procedure described in Example II(a); the experiment was repeated three times.

(b) The reaction mixtures obtained were subjected to a series of debromination reactions according to the procedure described in Example II(b) by means of the phosphites listed below.

The results of the HPLC analysis of the reaction mixtures obtained before and after debromination, are summarized in the following Table.

| | HPLC analysis ($10^{-3}$ mmoles/ml) | | | |
|---|---|---|---|---|
| Phosphorus compound | I | II | III | IV |
| Exp. 1. | | | | |
| – | – | 1.0 | – | 0.97 |
| tributyl phosphate | – | 2.0 | – | – |
| Exp. 2. | | | | |
| – | – | 2.3 | – | 0.47 |
| tributyl phosphite | 0.05 | 2.6 | – | – |
| Exp. 3. | | | | |
| – | 0.11 | 3.7 | 0.81 | 1.1 |
| tributyl phosphite | 0.83 | 4.6 | – | – |
| Exp. 4. | | | | |
| – | 0.26 | 3.3 | 0.59 | 0.66 |
| tristearyl phosphite | 0.04 | 4.0 | – | – |
| distearylpentaerythritol diphosphite | 0.79 | 4.0 | – | – |
| tris 25 phosphite | 0.77 | 3.8 | – | – |
| diphenyl didecyl(2,2,4-trimethyl-1,3-pentanediol) diphosphite | 0.73 | 4.1 | – | – |
| poly(dipropyleneglycol) phenyl phosphite | 0.96 | 3.7 | – | – |
| phenyl neopentylglycol phosphite | 0.78 | 3.7 | – | – |

(c) The reference compound III in the HPLC analysis of (b) above was prepared according to the process described in Example I(b); it can be characterized as follows:
NMR ($CDCl_3$): 1.57 (s, 9H), 2.22 (s, 3H), 5.18 (d, 1H, J Hz), 5.22 (s, 1H), 6.15 (dd, 1H, J 5 and 10 Hz), 7.22 (d, 1H, J 10 Hz), 8.31 (s, 1H).
IR: 3320, 1795, 1730, 1690, 1510, 1055 $cm^{-1}$.

(d) The reference compound IV in the HPLC analysis of (b) above was prepared as follows:

To a solution of 4.71 g (15 mmoles) of t-butyl-7-formamido-3-methyl-3-cephem-4-carboxylate 1-oxide in 250 ml of methylene chloride, cooled in ice and irradiated with a 150 W tungsten lamp, were added 3.15 ml (22.5 mmoles) of triethylamine and 2.7 g (15 mmoles) of N-bromosuccinimide. After irradiation for 10 minutes the starting material had disappeared completely, according to TLC. The dark-brown solution thus obtained was washed twice with dilute hydrochloric acid solution (pH 1) and then twice with water. After drying over magnesium sulphate and filtering, the solution was concentrated by evaporation to approximately 100 ml. Then 100 ml of acetic acid were added and the mixture was brominated as described hereinbefore with 2.7 g (15 mmoles) of N-bromosuccinimide. After irradiation for 1 hour, the conversion was not yet completed according to TLC. Again 1 g (5.6 mmoles) of N-bromosuccinimide was added and irradiation continued for 30 minutes. Ethyl acetate was then added and methylene chloride was removed by evaporation *in vacuo.* Then ice-water was added, the aqueous layer separated and remaining acetic acid removed by washing with sodium bicarbonate solution. The organic layer was dried over magnesium sulphate, filtered and concentrated by evaporation *in vacuo.* to approximately 100 ml. After removing some solid material by filtration, the residue was evaporated to dryness. The residue was treated with diethyl ether, petroleum ether was added and the product obtained by filtration. This resulted in 4.58 g (64.7% yield) of t-butyl 2-bromo-3-bromomethyl-7-formamido-3-cephem-4-carboxylate 1-oxide (reference compound IV).
NMR ($CDCl_3DMSO—D_6$): 1.57 (s, 9H), 4.35, 4.38, 4.61, 4.65 (ABq, 2H, J 10.5 Hz), 5.35 (d, 1H, J 5 Hz), 5.90 (s, 1H), 6.14 (dd, 1H, J 5 and 9 Hz), 8.28 (s, 1H). 8.46 (d, 1H, J 9Hz).
IR: 3280, 1805, 1730, 1690, 1510, 1060 $cm^{-1}$.

Example V

(a) A solution of t-butyl 7-phenylacetamido-3-methyl-3-cephem-4-carboxylate 1-oxide was brominated according to the procedure described in Example II(a); the experiment was repeated twice more.

(b) The reaction mixtures obtained were subjected to a series of debromination reactions according to the procedure described in Example II(b) by means of the phosphorus compounds mentioned below. The results of the HPLC analysis of the mixtures obtained before and after debromination are summarized in the following Tables.

| Phosphorus compound | HPLC analysis ($10^{-3}$ mmoles/ml) I | II | III | IV |
|---|---|---|---|---|
| Exp. 1. | | | | |
| — | — | 0.60 | 0.06 | 0.31 |
| methyl diphenyl-phosphinite | 0.05 | 0.93 | — | — |
| Exp. 2. | | | | |
| — | — | 3.1 | 0.28 | 0.69 |
| 2-ethoxy-4-methyl-1,3,2-dioxaphospholane | 0.30 | 3.6 | — | — |
| 4-methyl-2-piperidino-1,3,2-dioxaphospholane | 0.30 | 3.5 | — | — |

| Phosphorus compound | in mg/ml | HPLC analysis (10$^{-3}$ mmoles/ml) | | | |
|---|---|---|---|---|---|
| | | I | II | III | IV |
| Exp. 3. | | | | | |
| — | — | — | 2.2 | 0.19 | 0.45 |
| triallyl phosphite | 0.72 | 0.21 | 2.6 | — | — |
| triallyl phosphite | 1.6 | 0.21 | 2.6 | — | — |

This last Experiment demonstrates that a large excess of debrominating agent may be used without affecting the net result.

Example VI

(a) A solution of 2,2,2-trichloroethyl 7-formamido-3-methyl-3-cephem-4-carboxylate 1-oxide was brominated according to the procedure described in Example II(a); the experiment was repeated three times.

(b) The reaction mixtures obtained were subjected to a series of debromination reactions according to the procedure described in Example II(b) by means of the phosphorus compounds mentioned below. The results of the HPLC analysis of the mixtures before and after debromination are summarized in the following Table.

| Phosphorus compound | HPLC analysis (10$^{-3}$ mmoles /ml) | | | |
|---|---|---|---|---|
| | I | II | III | IV |
| Exp. 1. | | | | |
| – | 0.07 | 1.9 | 0.24 | 0.49 |
| trimethyl phosphite | 0.31 | 2.3 | – | – |
| triisooctyl phosphite | 0.31 | 2.3 | – | – |
| triphenyl phosphite | 0.36 | 2.3 | – | – |
| diphenyl isodecyl phosphite | 0.31 | 2.3 | – | – |
| Exp. 2. | | | | |
| – | – | 1.5 | – | 1.1 |
| triallyl phosphite | – | 2.5 | – | – |
| tributyl phosphite | – | 2.5 | – | – |
| diphenyl 2-ethylhexyl phosphite | – | 2.5 | – | – |
| triisodecyl phosphite | – | 2.6 | – | – |
| methyl diphenyl-phosphinite | – | 2.5 | – | – |
| Exp. 3. | | | | |
| – | 0.10 | 1.4 | – | 0.36 |
| triethyl phosphite | 0.10 | 1.8 | – | – |
| diphenyl 2-ethylhexyl phosphite | 0.10 | 1.8 | – | – |

| Phosphorus compound | HPLC analysis ($10^{-3}$ mmoles/ml) | | | |
|---|---|---|---|---|
| | I | II | III | IV |
| Exp. 3. | | | | |
| diphenyl isooctyl phosphite | 0.10 | 1.8 | – | – |
| hexamethylphosphorous triamide | 0.10 | 1.6 | – | – |
| Exp. 4. | | | | |
| | 0.05 | 2.6 | 0.36 | 0.82 |
| diethyl phenyl-phosphonite | 0.39 | 3.3 | – | – |
| 2-ethoxy-4-methyl-1,3,2-dioxaphospholane | 0.46 | 3.2 | – | – |
| diethyl N-methyl-N-phenyl-phosphoramidite | 0.46 | 3.3 | – | – |

(c) The reference compound III in the HPLC analysis of (b) was prepared according to the procedure described in Example I(b); it can be characterized as follows:
NMR ($CDCl_3$): 2.34 (s, 3H), 4.96 (s, 2H), 5.25 (d, 1H, J 4.5 Hz), 5.29 (s, 1H), 6.22 (dd, 1H, J 4.5 and 10 Hz), 7.17 (s, 1H,, J 10 Hz), 8.33 (s, 1H).
IR: 3320, 1800, 1745, 1690, 1510, 1050 $cm^{-1}$.

(d) The reference compound IV in the HPLC analysis of (b) was prepared according to the procedure described in Example IV(d); it can be charcterized as follows:
NMR ($CDCl_3$): 4.21, 4.39, 4,68, 4.86 (ABq, 2H, J 11 Hz), 4.82, 5.02, 5.03, 5.24 (ABq, 2H, J 12 Hz), 5.08 (d, 1H, J 5 Hz), 5.75 (s, 1H), 6.24 (dd, 1H, J 5 and 10 Hz), 7.57 (d, 1H, J 10 Hz), 8.32 (s, 1H).
IR: 3270, 1810, 1750, 1690, 1505, 1055 $cm^{-1}$.

## Example VII

(a) A solution of t-butyl 7-benzamido-3-methyl-3-cephem-4-carboxylate 1-oxide was brominated according to the procedure described in Example II(a); the experiment was repeated four times.

(b) The reaction mixtures obtained were subjected to a series of debromination reactions according to the procedure described in Example II(b) by means of the phosphorus compounds listed below. The results of the HPLC analysis of the reaction mixtures obtained before and after debromination are summarized in the following Table.

| Phosphorus compound | HPLC analysis ($10^{-3}$ mmoles /ml) | | | |
|---|---|---|---|---|
| | I | II | III | IV |
| Exp. 1. | | | | |
| – | – | 1.7 | 0.19 | 1.8 |
| 2-ethylhexyl dioctyl phosphite | 0.17 | 3.2 | – | – |
| tris(2-chloroethyl) phosphite | 0.17 | 3.2 | – | – |
| diphenyl isooctyl phosphite | 0.17 | 3.2 | – | – |
| Exp. 2. | | | | |
| – | – | 2.0 | 0.31 | 1.1 |
| triallyl phosphite | 0.31 | 3.1 | – | – |
| Exp. 3. | | | | |
| – | – | – | – | 1.8 |
| chlorodiphenylphosphine | – | 1.0 | – | 0.16 |
| Exp. 4. | | | | |
| – | – | 0.67 | 0.07 | 1.0 |
| benzyl diethyl phosphite | 0.07 | 1.7 | – | – |
| tri-o-tolyl phosphite | 0.06 | 1.5 | – | – |
| trilauryl phosphite | 0.05 | 1.5 | – | – |
| diisodecyl pentaerythritol diphosphite | 0.06 | 1.6 | – | – |
| tetraphenyl dipropylene-glycol diphosphite | 0.06 | 1.6 | – | – |
| tris(dipropyleneglycol) phosphite | 0.09 | 1.6 | – | – |
| tetrakis(nonylphenyl)poly-propyleneglycol diphosphite | 0.05 | 1.6 | – | – |

| Phosphorus compound | HPLC analysis ($10^{-3}$ mmoles/ml) | | | |
|---|---|---|---|---|
| | I | II | III | IV |
| Exp. 5. | | | | |
| – | – | 1.2 | 0.20 | 0.46 |
| heptakis(dipropyleneglycol) triphosphite | 0.24 | 1.6 | – | – |
| tri(p-chlorophenyl) phosphite | 0.20 | 1.6 | – | – |

The preparation of the various reference compounds used in the HPLC analysis has been described in Example I.

Example VIII

(a) A solution of methyl 7-phenylacetamido-3-methyl-3-cephem-4-carboxylate 1-oxide was brominated according to the procedure described in Example II(a).

(b) The reaction mixture was subjected to a series of debromination reactions according to the procedure described in Example II(b) by means of the phosphites listed below. The results of the HPLC analysis of the reaction mixtures obtained before and after debromination are summarized in the following Table.

| Phosphorus compound | HPLC analysis ($10^{-3}$ mmoles/ml) | | | |
|---|---|---|---|---|
| | I | II | III | IV |
| – | 0.08 | 1.2 | 0.20 | 0.23 |
| trimethyl phosphite | 0.28 | 1.4 | – | – |
| triallyl phosphite | 0.28 | 1.4 | – | – |
| diphenyl 2-ethylhexyl phosphite | 0.28 | 1.4 | – | – |

(c) The reference compound III in the HPLC analysis of (b) was prepared according to the procedure described in Example I(b); it can be characterized as follows:
NMR ($CDCl_3$): 2.17 (s, 3H), 3.59 (s, 2H), 3.85 (s, 3H), 5.11 (d, 1H, J 4.8 Hz), 5.12 (s, 1H), 6.08 (dd, 1H, J 4.8 and 9.5 Hz), 6.92 (d, 1H, J 9.5 Hz), 7.30 (s, 5H).
IR: 3300, 1795, 1735, 1680, 1515, 1055 $cm^{-1}$.

(d) The reference compound IV in the HPLC analysis of (b) was prepared as follows:

1. 1 g (1.8 mmol) of t-butyl 2-bromo-3-bromomethyl-7-phenylacetamido-3-cephem-4-carboxylate 1-oxide [prepared according to the procedure described in Example II(c)] was dissolved in 15 ml of trifluoroacetic acid. After standing for 15 minutes at room temperature, the solution was evaporated to dryness. The residue was dissolved in methylene chloride and the solution was again evaporated to dryness. The residue was treated with diethyl ether and the product isolated by filtration and washed with diethyl ether and hexane. There was obtained 0.8 g (88% yield) of 2-bromo-3-bromomethyl-7-phenylacetamido-3-cephem-4-carboxylic acid 1-oxide.
NMR ($CF_3COOD$): 3.98 (s, 2H), 4.12, 4.30, 4.81, 4,99 (ABq, 2H, J 11 Hz), 5.56 (d, 1H, J 5 Hz), 5.92 (s, 1H), 6.34 (d, 1H, J 5 Hz), 7.32 (s, 5H).
IR: 3310, 2950, 1780, 1720, 1700, 1520, 1050 $cm^{-1}$.

2. To a solution of the product thus obtained in 20 ml of tetrahydrofuran was added an excess of diazomethane in diethyl ether. When the nitrogen generation was completed, the excess diazomethane was destroyed with acetic acid. The solution was then evaporated to dryness and the residue dissolved in some methylene chloride. The product precipitated upon addition of diethyl ether and hexane. The product was collected by filtration and washed with hexane. There was obtained 0.63 g (77% yield) of methyl 2-bromo-3-bromomethyl-7-phenylacetamido-3-cephem-4-carboxylate 1-oxide (compound IV). NMR ($CDCl_3$): 3.60 (s, 2H), 3.89 (s, 3H), 4.15, 4.63 (ABq, 2H, J 10.5 Hz), 5.13 (d, 1H, J 5 Hz), 5.51 (s, 1H), 6.16 (dd. 1H, J 5 and 10 Hz), 6.70 (d, 1H, J 10 Hz), 7.25 (s, 5H).
IR: 3395, 1805, 1735, 1680, 1510, 1045 $cm^{-1}$.

Example IX

(a) A solution of t-butyl 7-phenoxyacetamido-3-methyl-3-cephem-4-carboxylate 1-oxide was brominated according to the procedure described in Example II(a); the experiment was repeated.

(b) The reaction mixtures were subjected to a series of debromination reactions according to the procedure described in Example II(b) by means of the phosphites listed below. The results of the HPLC analysis of the reaction mixtures obtained before and after debromination are summarized in the following Table.

| | HPLC analysis ($10^{-3}$ mmoles /ml) | | |
|---|---|---|---|
| Phosphorus compound | I | II | III |
| Exp. 1. | | | |
| – | – | 0.88 | 0.10 |
| triisooctyl phosphite | 0.10 | 1.4 | – |
| triisodecyl phosphite | 0.10 | 1.4 | – |
| tributyl phosphite | 0.10 | 1.4 | – |
| Exp. 2. | | | |
| – | – | 1.1 | 0.04 |
| diphenyl isodecyl phosphite | 0.04 | 1.4 | – |
| diphenyl isooctyl phosphite | 0.05 | 1.4 | – |
| triisodecyl phosphite | 0.05 | 1.4 | – |

(c) The reference compound III in the HPLC analysis of (b) was prepared according to the procedure described in Example I(b); it can be characterized as follows:
NMR ($CDCl_3$): 1.56 (s, 9H), 2.19 (s, 3H), 4.57 (s, 2H), 5.14 (s, 1H), 5.16 (d, 1H, J 4.5 Hz), 6.16 (dd, 1H, J 4.5 and 10 Hz), approximately 6.78—7.50 (m, 5H), 7.82 (d, 1H, J 10 Hz).
IR: 3370, 1800, 1730, 1700, 1520, 1055 $cm^{-1}$.

(d) The comments in relation to reference compound IV at the end of Example III apply to the present reference compound IV as well.

Example X

(a) A solution of 2,2,2-trichloroethyl 7-phenoxy-acetamido-3-methyl-3-cephem-4-carboxylate 1-oxide was brominated according to the procedure described in Example II(a); the experiment was repeated.

(b) the reaction mixture was subjected to a series of debromination reactions according to the procedure described in Example II(b) by means of the phosphites listed below. The results of the HPLC analysis of the reaction mixtures obtained before and after debromination are summarized in the following Table.

| Phosphorus compound | HPLC analysis ($10^{-3}$ mmoles/ml) | | |
|---|---|---|---|
| | I | II | III |
| Exp. 1. | | | |
| – | 0.15 | 0.79 | 0.39 |
| 2-ethylhexyl dioctyl phosphite | 0.57 | 1.0 | – |
| tris (2-chloroethyl) phosphite | 0.57 | 1.1 | – |
| diphenyl isodecyl phosphite | 0.55 | 1.0 | – |
| triethyl phosphite | 0.57 | 1.0 | – |
| Exp. 2. | | | |
| – | – | 1.0 | 0.10 |
| 2-ethylhexyl dioctyl phosphite | 0.12 | 1.3 | – |
| triisooctyl phosphite | 0.12 | 1.3 | – |
| tributyl phosphite | 0.12 | 1.3 | – |

(c) The reference compound III in the HPLC analysis of (b) was prepared according to the procedure described in Example I(b); it can be characterized as follows:
NMR ($CDCl_3$): 2.30 (s, 3H), 4.56 (s, 2H), 4.94 (s, 2H), 5.18 (s, 1H), 5.22 (d, 1H, J 4.5 Hz), 6.23 (dd, 1H, J 4.5 and 10.5 Hz), approximately 6.77—7.50 (m, 5H), 7.81 (d, 1H, J 10.5 Hz).
IR: 3320, 1800, 1745, 1695, 1520, 1055 $cm^{-1}$.

(d) The comments in relation to reference compound IV at the end of Example III apply to the present reference compound IV as well.

## Example XI

(a) A solution of benzhydryl 7-phenylacetamido-3-methyl-3-cephem-4-carboxylate 1-oxide was brominated according to the procedure described in Example II(a), the experiment was repeated twice more.

(b) The reaction mixtures obtained were subjected to a series of debromination reactions according to the procedure described in Example II(b) by means of the phosphorus compounds listed below. The results of the HPLC analysis of the reaction mixtures obtained before and after debromination are summarized in the following Table.

| Phosphorus compound | HPLC analysis (10⁻³ mmoles/ml) | | |
|---|---|---|---|
| | I | II | III |
| Exp. 1. | | | |
| – | – | 1.2 | 0.03 |
| tributyl phosphite | 0.07 | 1.4 | – |
| triallyl phosphite | 0.07 | 1.4 | – |
| 2-ethoxy-4-methyl-1,3,2-dioxaphospholane | 0.07 | 1.3 | – |
| Exp. 2. | | | |
| – | – | 1.2 | 0.17 |
| tributyl phosphite | 0.20 | 1.7 | – |
| triisodecyl phosphite | 0.20 | 1.8 | – |
| Exp. 3. | | | |
| – | 0.27 | 1.6 | 0.14 |
| triethyl phosphite | 0.47 | 1.8 | – |
| diphenyl 2-ethylhexyl phosphite | 0.45 | 1.8 | – |

(c) The reference compound III in the HPLC analysis of (b) was prepared according to the procedure described in Example I(b); it can be characterized as follows:
NMR ($CDCl_3$): 2.14 (s, 3H), 3.63 (s, 2H), 5.14 (d, 1H, J 4.5 Hz), 5.17 (s, 1H), 6.08 (dd, 1H, J 4.5 and 10 Hz), 6.96 (s, 1H), approximately 7.15—7.63 (m, 16H).
IR: 3360, 1790, 1735, 1685, 1520, 1050 $cm^{-1}$.

(d) The comments in relation to reference compound IV at the end of Example III apply to the present reference compound IV as well.

Example XII

(a) A solution of benzyl 7-benzamido-3-methyl-3-cephem-4-carboxylate 1-oxide was brominated according to the procedure described in Example II(a).

(b) The reaction mixture obtained was subjected to debromination by means of the phosphites mentioned below. The results of the HPLC analysis obtained before and after the bromination has been carried out can be summarized in the usual manner.

| Phosphorus compound | HPLC analysis ($10^{-3}$ mmoles/ml) | |
|---|---|---|
| | I | III |
| – | – | 2.0 |
| tributyl phosphite | 2.0 | – |
| triallyl phosphite | 2.0 | – |

(c) The reference compound III in the HPLC analysis of (b) was prepared according to the procedure described in Example I(b); it can be characterized as follows:
NMR ($CDCl_3$): 2.23 (s, 3H), 5.19 (s, 1H), 5.24 (d, 1H, J 4.8 Hz), 5.31 (s, 2H), 6.32 (dd, 1H J 4.8 and 10 Hz), approximately 7.22—8.12 (m, 11H).
IR: 3400, 1795, 1730, 1670, 1520, 1050 $cm^{-1}$.

(d) The starting material in the processes of (a) and (c), i.e. benzyl 7-benzamido-3-methyl-3-cephem-4-carboxylate 1-oxide, can be prepared according to the procedure described in Example I(a); it can be characterized as follows:
NMR (DMSO—$D_6$ in pyridine-$D_5$ 2:1): 2.12 (s, 3H), 3.93 (br., 2H), 5.27 (d, 1H, J 4.5 Hz), 5.44 (s, 2H), 6.36 (dd, 1H, J 4.5 and 9.5 Hz), 7.24—8.14 (m, 5H), 8.78 (d, 1H, J 9.5 Hz).
IR: 3300, 1780, 1720, 1645, 1530, 1035 $cm^{-1}$.

Example XIII

(a) The procedure of Example XII(a) was repeated using 4-nitrobenzyl 7-phenylacetamido-3-methyl-3-cephem-4-carboxylate 1-oxide as the starting material.

(b) the results of the following debromination procedure are summarized in the following Table.

| Phosphorus compound | HPLC analysis ($10^{-3}$ mmoles/ml) | |
|---|---|---|
| | I | III |
| – | 0.04 | 2.2 |
| trimethyl phosphite | 2.5 | – |
| triethyl phosphite | 2.5 | – |
| triallyl phosphite | 2.5 | – |
| tributyl phosphite | 2.5 | – |

(c) The reference compound III in the HPLC analysis of (b) was prepared according to the procedure described in Example I(b); it can be characterized as follows:
NMR ($CDCl_3$): 2.20 (s, 3H), 3.61 (s, 2H), 5.12 (s, 1H), 5.13 (d, 1H, J 4.5 Hz), 5.35 (s, 2H), 6.11 (dd, 1H, J 4.5 and 9.7 Hz), 6.85 (d, 1H, J 9.7 Hz), 7.31 (s, 5H), 7.50, 7.64, 8.15, 8.30 (ABq, 4H, J 9 Hz).
IR: 3310, 1795, 1730, 1675, 1525, 1350, 1055 $cm^{-1}$.

(d) The starting material in the processes of (a) and (c), i.e. 4-nitrobenzyl 7-phenylacetamido-3-methyl-3-cephem-4-carboxylate 1-oxide, can be prepared according to the procedure described in Example I(a); it can be characterized as follows:
NMR ($CF_3CO_2D$): 2.28 (s, 3H), 3.47, 3.81, 3.87 (s, 2H), 3.95, 4.28 (ABq, 2H, J 20 Hz), 5.03 (d, 1H, J 4 Hz), 5.55 (s, 2H), 6.23 (d, 1H, J 4 Hz), 7.34 (s, 5H), 7.60 7.75, 8.23 8.47 (ABq, 4H, J 8.5 Hz).
IR: 3280, 1770, 1730, 1645, 1525, 1350, 1035 $cm^{-1}$.

Example XIV

(a) The procedure of Example XII(a) was repeated using 4-methoxybenzyl 2-bromo-7-phenylaceamido-3-methyl-3-cephem-4-carboxylate 1-oxide as the starting material.

(b) The results of the following debromination procedure are summarized in the following Table.

| Phosphorus compound | HPLC analysis ($10^{-3}$ mmoles /ml) | |
|---|---|---|
| | I | III |
| – | – | 1.9 |
| trimethyl phosphite | 2.0 | – |
| triethyl phosphite | 2.0 | – |
| triallyl phosphite | 2.0 | – |
| tributyl phosphite | 2.0 | – |

(c) The reference compound III in the HPLC analysis of (b) was prepared according to the procedure described in Example I(b); it can be characterized as follows:
NMR ($CDCl_3$): 2.15 (s, 3H), 3.59 (s, 2H), 3.77 (s, 3H), 5.05 (d, 1H, J 4.8 Hz), 5.07 (s, 1H), 6.05 (dd, 1H, J 4.8 and 10 Hz), 6.81, 6.95, 7.26, 7.40 (ABq, 4H, J 9 Hz), 6.85 (d, 1H, J 10 Hz), 7.29 (s, 5H).
IR: 3320, 1790, 1725, 1670, 1520, 1055 $cm^{-1}$.

Example XV

(a) A solution of t-butyl 2-bromo-3-bromomethyl-7-formamido-3-cephem-4-carboxylate 1-oxide was prepared according to the procedure described in Example IV(d).

(b) The results of the following debromination procedure are summarized in the following Table.

| Phosphorus compound | HPLC analysis ($10^{-3}$ mmoles /ml) | |
|---|---|---|
| | II | IV |
| – | – | 5.1 |
| trimethyl phosphite | 4.7 | – |
| triethyl phosphite | 4.8 | – |
| tributyl phosphite | 4.8 | – |

Example XVI

(a) The procedure of Example XV(a) was repeated using t-butyl 7-benzamido-3-methyl-3-cephem-4-carboxylate 1-oxide as a starting material.

(b) The results of the following debromination procedure are summarized in the following Table.

| Phosphorus compound | HPLC analysis ($10^{-3}$ mmoles/ml) | |
|---|---|---|
| | II | IV |
| – | – | 2.8 |
| trimethyl phosphite | 2.6 | – |
| triethyl phosphite | 2.6 | – |
| tributyl phosphite | 2.6 | – |

(c) The preparation of the reference compound IV has been described in Example I(c).

Example XVII

In order to establish the influence of the solvent on the debromination part of the process of the invention a particular debromination reaction was studied using a range of different solvents.

In this procedure a solid mixture of bromo derivatives of t-butyl 7-phenylacetamido-3-methyl-3-cephem-4-carboxylate 1-oxide was dissolved in acetic acid. To 2 ml of this solution were added 20 ml of one of the solvents mentioned below and 0.1 ml of tributyl phosphite; then solvent was added until the total volume was 25 ml. Accordingly, the solutions thus prepared contained each 8% of acetic acid.

The results of the HPLC analysis of the various experiments are listed below.

| Solvent | HPLC analysis (10$^{-3}$ mmoles/ml) | | | |
|---|---|---|---|---|
| | I | II | III | IV |
| Exp. 1. | | | | |
| acetone (blank) | – | 1.6 | 0.12 | 0.23 |
| acetonitrile | 0.16 | 1.9 | – | – |
| acetic acid | 0.15 | 1.8 | – | – |
| N,N-dimethylacetamide | 0.15 | 1.8 | – | – |
| dioxane | 0.13 | 1.8 | – | – |
| acetone | 0.16 | 1.9 | – | – |
| 2-methoxyethanol | 0.18 | 1.7 | – | – |
| Exp. 2. | | | | |
| acetone (blank) | 0.03 | 2.4 | 0.57 | 0.39 |
| propylene carbonate | 0.64 | 2.7 | – | – |
| ethyl acetate | 0.62 | 2.8 | – | – |
| tetrahydrofuran | 0.62 | 2.8 | – | – |
| dimethoxyethane | 0.64 | 2.9 | – | – |

It may be concluded that the debromination process proceeds quantitatively in various classes of solvents.

Example XVIII

The procedure of Example XVII was repeated but t-butyl 7-benzamido-3-methyl-3-cephem-4-carboxylate 1-oxide was used as the starting material.

The results of the HPLC analysis of the debromination process in still some other solvents are listed below.

| Solvents | HPLC analysis (10$^{-3}$ mmoles/ml) | | | |
|---|---|---|---|---|
| | I | II | III | IV |
| acetone (blank) | – | – | 0.66 | 1.8 |
| bis(2-methoxyethyl)ether | 0.54 | 1.6 | – | – |
| tetrahydrothiophene 1,1-dioxide | 0.49 | 1.5 | – | – |

Example XIX

(a) To a solution of 10.1 g (25 mmoles) of t-butyl 7-phenylacetamido-3-methyl-3-cephem-4-carboxylate 1-oxide in 400 ml of dry methylene chloride, which was kept under nitrogen and cooled in an ice bath, were added 6.7 g (37.5 mmoles) of N-bromosuccinimide while the mixture was irradiated with a 150 W tungsten lamp. The irradiation was continued for 2.25 hours and then the reaction mixture was washed with water and dried over magnesium sulphate. After evaporation to a small volume, the products were precipitated by addition of diethyl ether. There were obtained 7.6 g of a solid substance.

(b) 625 mg of the solid thus obtained were dissolved in 50 ml of acetone. 4 ml of this solution were diluted with acetone to 25 ml and subjected to HPLC analysis.

To other samples of each 4 ml of the solution, one of the brominating agents mentioned below was added in approximately equimolar quantities. The sodium sulphite and sodium dithionite were dissolved in water, the stannous chloride in 4 N HCl solution. The results of the HPLC analysis before and after the debromination reactions are summarized in the following Table.

| Debromination agent | HPLC analysis ($10^{-3}$ mmoles /ml) | | | |
|---|---|---|---|---|
| | I | II | III | IV |
| Exp. 1. | | | | |
| — | — | 2.6 | 0.21 | 0.56 |
| stannous chloride | — | 3.2 | — | — |
| Exp. 2. | | | | |
| — | — | 2.7 | 0.21 | 0.56 |
| sodium sulphite | 0.24 | 3.3 | — | — |
| Exp. 3. | | | | |
| — | — | 2.6 | 0.21 | 0.58 |
| sodium dithionite | 0.22 | 3.1 | — | — |

Example XX

(a) 5.6 g of t-butyl 2-bromo-3-bromoethyl-7-phenylacetamido-3-cephem-4-carboxylate 1-oxide, prepared according to the procedure described in Example II(c), were dissolved in 40 ml of trifluoroacetic acid and the solution was stirred for 15 minutes at room temperature. The solvent was then evaporated, diethyl ether was added and the solvent evaporated again. Diethyl ether was added again; the solid was filtrated and dried under vacuum at room temperature. There were obtained 4.72 g (93.6% yield) of 2-bromo-3-bromomethyl-7-phenylacetamido-3-cephem-4-carboxyic acid 1-oxide as a pale yellow solid.

NMR (DMSO—D6): 3.60 (s, 2H), 4.46 (s, 2H), 5.29 (d, 1H, J 4.5 Hz) 5.87 (dd, 1H, J 4.5 and 7.5 Hz), 6.09 (s, 1H), 7.20 (s, 5H), 8.53 (d, 1H, J 7.5 Hz).

IR: 3340, 1790, 1730, 1705, 1620, 1525, 1040 $cm^{-1}$.

(b) 230.0 mg (0.45 mmoles) of the products of (a) were suspended in 2.5 ml of chloroform. Then 318 mg (1.32 mmoles) of trimethylsilyl trimethylsilylamidosulphonate were added and the mixture was stirred for 4 hours at 40°C under nitrogen. According to the NMR spectrum the solution contained trimethylsilyl 2-bromo-3-bromomethyl-7-phenylacetamido-3-cephem-4-carboxylate 1-oxide.

NMR ($CDCl_3$): 0.33 (s, 9H), 3.65 (s, 2H), 4.23, 5.05 (ABq, 2H, J 10.5 Hz), 5.22 (d, 1H, J 5Hz), 5.62 (s, 1H), 6.18 (dd, 1H, J 5 and 9.5 Hz), 6.96 (d, 1H, J 9.5 Hz), 7.3 (s).

(c) To half of the solution of (b) 55 mg of trimethyl phosphite were added. The NMR spectrum demonstrated clearly that debromination had taken place and that trimethylsilyl 3-bromomethyl-7-phenylacetamido-3-cephem-4-carboxylate 1-oxide was formed *in situ.*

NMR ($CHCl_3$): 0.35 (s, 9H), approximately 3.2—4.2 (m), 4.30, 4.77 (ABq, 2H, J 10.5 Hz), 4.63 (d, 1H, J 5Hz), 6.05 (dd, 1H, J 5 and 9.5 Hz), 7.05 (d, 1H, J 9.5 Hz), 7.4 (s).

Example XXI

(a) A quantity of approximately 0.2 mmoles of 2-bromo-3-bromomethyl-7-phenylacetamido-3-cephem-4-carbxylic acid 1-oxide was suspended in 10 ml of 1,2-dichloroethane. After the addition of about 0.7 mmoles of trimethylsilyl trimethylsilylamidosulphonate the mixture was stirred for 1.5 hours at 30°C under nitrogen. According to the NMR spectrum the reaction mixture contained trimethylsilyl 2-bromo-3-bromomethyl-7-phenylacetamido-3-cephem-4-carboxylate 1-oxide.

(b) The reaction mixture obtained was subjected to a series of debromination reactions by means of the phosphites mentioned below. Before the HPLC analysis was carried out the trimethylsilyl 3-bromomethyl-7-phenylacetamido-3-cephem-4-carboxylate 1-oxide thus prepared was first converted into the corresponding methyl ester.

This was effected by adding each of the reaction mixtures obtained after the series of debromination reactions was carried out, to an excess of a solution of diazomethane in diethyl ether containing some methanol. When nitrogen was no longer evolved, the excess of diazomethane was destroyed with acetic acid and the solvent was evaporated. The residue was dissolved in acetone and the volume of the solution was adjusted to 250 ml. This solution was then used in the HPLC analysis as described previously.

This procedure was repeated with another portion of the reaction mixture obtained according to (a), thus producing a solution of methyl 2-bromo-3-bromomethyl-7-phenylacetamido-3-cephem-4-carboxylate 1-oxide.

The results of the HPLC analysis of these solutions of methyl esters, corresponding to the trimethylsilyl derivatives obtained before and after the debromination reactions were carried out, are summarized in the Table below.

| Phosphorus compound | HPLC analysis ($10^{-3}$ mmoles/ml) | |
|---|---|---|
| | II | IV |
| Exp. 1. | | |
| — | — | 0.60 |
| tributyl phosphite | 0.58 | — |
| Exp. 2. | | |
| — | — | 0.56 |
| triisooctyl phosphate | 0.52 | — |
| Exp. 3. | | |
| — | — | 0.54 |
| triallyl phosphite | 0.62 | — |
| Exp. 4. | | |
| — | — | 0.46 |
| trimethyl phosphite | 0.47 | — |
| Exp. 5. | | |
| — | — | 0.56 |
| benzyl diethyl phosphite | 0.55 | — |
| Exp. 6. | | |
| — | — | 0.68 |
| bis-o-phenylene pyrophosphite | 0.60 | — |

Example XXII

Trimethylsilyl 7-phenylacetamido-3-methyl-3-cephem-4-carboxylate 1-oxide was brominated according to the process described in Example II (a) using 1,2-dichloroethane as a solvent. The reaction mixture was subjected to a debromination reaction according to the procedure described in Example II (b).

The conversion of the products into corresponding methyl esters, before and after the debromination reaction was carried out, was effected as described in Example XXI.

The results of the HPLC analysis of the solutions of methyl esters thus obtained are summarized in the Table below.

| | HPLC analysis ($10^{-3}$ mmoles/ml) | | | |
|---|---|---|---|---|
| Phosphorus compound | I | II | III | IV |
| – | – | 0.23 | 0.04 | 0.07 |
| tributyl phosphite | 0.03 | 0.30 | – | – |

## Claims

1. Improved process for the preparation of 3-bromomethyl-3-cephem sulphoxide derivatives of the general formula

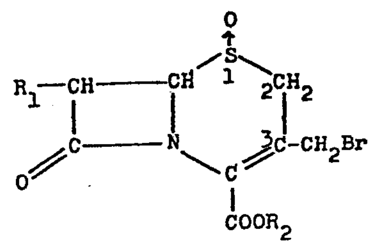

I

wherein $R_1$ represents an acylamido group and $R_2$ represents a group protecting the carboxy radical, by brominating in manner known *per se* a desacetoxy-3-cephem sulphoxide of the general formula:

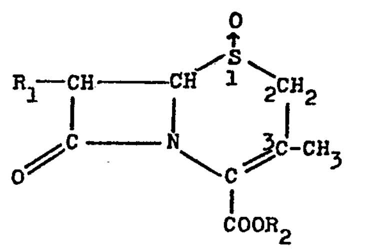

II

wherein $R_1$ and $R_2$ are as hereinbefore defined, to convert *inter alia* the methyl group in the 3-position of such a compound to a bromomethyl group, characterized by treating the reaction mixture that is obtained after the bromination has been effected, containing a compound of the general formula:

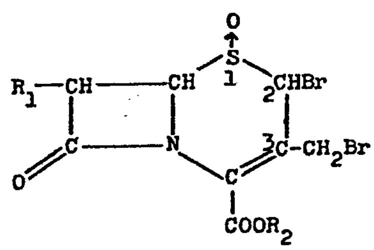

III

wherein $R_1$ and $R_2$ are as hereinbefore defined, or optionally a compound of general formula III separated from such a reaction mixture, with a debrominating agent selected from the group consisting of trivalent organic phosphorus compounds and inorganic salts which are capable of replacing—in the presence of a hydrogen donor—the bromine atom in the 2-position by hydrogen, and recovering by methods known *per se* the 3-bromomethyl-3-cephem sulphoxide derivative of general formula I thus produced.

2. Process according to claim 1, wherein the trivalent organic phosphorus compound is selected from the group consisting of

(a) phosphites of the general formula:

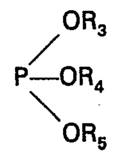 IV

wherein $R_3$, $R_4$ and $R_5$ are the same or different and each represents a straight- or branched-chain alkyl group which may be substituted by a chlorine atom and has from 1 to 18 carbon atoms, a phenyl group (which may be substituted by a methyl group or a chlorine atom), an allyl or benzyl group;

(b) other phosphites, for example phenyl neopentylglycol phosphite, poly(dipropyleneglycol) phenyl phosphite, tris 25 phosphite and tris(dipropyleneglycol) phosphite;

(c) di- and triphosphites, for example diisodecyl pentaerythritol diphosphite, tetraphenyl dipropyleneglycol diphosphite, distearyl pentaerythritol diphosphite, diphenyl didecyl (2,2,4-trimethyl-1,3-pentanediol) diphosphite, tetrakis (nonylphenyl) polypropyleneglycol diphosphite and heptakis (dipropyleneglycol) triphosphite;

(d) phosphonites, for example di(lower alkyl) phenylphosphonites;

(e) phosphinites, for example lower alkyl diphenylphosphinites;

(f) phosphines, for example chlorodiphenylphosphine;

(g) other trivalent phosphorus compounds, for example hexamethylphosphorous triamide, 2-ethoxy-4-methyl-1,3,2-dioxaphospholane, 4-methyl-2-piperidino-1,3,2-dioxaphospholane, diethyl N-methyl-N-phenylphosphoramidite and bis-o-phenylene pyrophosphite;

3. Process according to claim 1, wherein the inorganic salt is stannous chloride, sodium sulphite or sodium dithionite.

4. Process according to claim 1 or 2, wherein the quantity of the debrominating agent is in excess in relation to the quantity of the compound of formula III.

5. Process according to claim 1 or 3, wherein the debrominating agent is used in equimolar quantity in relation to the compound of formula III.

6. Process according to claim 2, wherein the trivalent organic phosphorus compound is trimethyl phosphite, triethyl phosphite, tripropyl phosphite, triisopropyl phosphite, tributyl phosphite, triisobutyl phosphite, triamyl phosphite, trioctyl, phosphite, triisooctyl phosphite, tris (2-ethylhexyl) phosphite, tridecyl phosphite, triisodecyl phosphite, trineodecyl phosphite, trilauryl phosphite, tristearyl phosphite, triallyl phosphite, tris(2-chloroethyl) phosphite, triphenyl phosphite, tris(p-methylphenyl) phosphite, tris-o-tolyl phosphite, tris(p-chlorophenyl) phosphite, 2-ethylhexyl dioctyl phosphite, diethyl benzyl phosphite, didecyl benzyl phosphite, diisodecyl phenyl phosphite, isooctyl diphenyl phosphite, 2-ethylhexyl diphenyl phosphite, decyl diphenyl phosphite, isodecyl diphenyl phosphite, dimethyl phenylphosphonite, diethyl phenylphosphonite, di(t-butyl) phenylphosphonite, methyl diphenylphosphinite or ethyl diphenylphosphinite.

7. Process according to claim 2, wherein the trivalent organic phosphorus compound is trimethyl phosphite, triethyl phosphite, tributyl phosphite, triisooctyl phosphite, triisodecyl phosphite, trilauryl phosphite, tristearyl phosphite, triallyl phosphite, tris(2-chloroethyl) phosphite, triphenyl phosphite, tris-o-tolyl phosphite, tris(p-chlorophenyl) phosphite, 2-ethylhexyl dioctyl phosphite, diethyl benzyl phosphite, isooctyl diphenyl phosphite, 2-ethylhexyl diphenyl phosphite, isodecyl diphenyl phosphite, phenyl neopentylglycol phosphite, poly(dipropyleneglycol) phenyl phosphite, tris 25 phosphite, tris (dipropyleneglycol) phosphite, diisodecyl pentaerythritol diphosphite, tetraphenyl dipropyleneglycol diphosphite, distearyl pentaerythritol diphosphite, diphenyl didecyl (2,2,4-trimethyl-1,3-pentanediol) diphosphite, tetrakis(nonylphenyl) polypropyleneglycol diphosphite, heptakis(dipropyleneglycol) triphosphite, bis-o-phenylene pyrophosphite, hexamethylphosphorous triamide, 2-ethoxy-4-methyl-1,3,2-dioxaphospholane, 4-methyl-2-piperidino-1,3,2-dioxaphospholane, diethyl N-methyl-N-phenylphosphoramidite, diethyl phenylphosphonite, methyl diphenylphosphinite or chlorodiphenylphosphine.

8. Process according to claim 1, wherein the hydrogen donor is water, a lower alkanol, for example, methanol or a carboxylic acid, for example, acetic acid.

9. Process according to claim 1, wherein $R_1$ represents phenylacetamido, phenoxyacetamido, benzamido or formamido.

10. Process according to claim 1, wherein $R_2$ represents a straight- or branched-chain alkyl group having from 1 to 4 carbon atoms optionally substituted by one or more halogen atoms or by one or two phenyl groups, which phenyl groups may themselves carry one or more substituents for example nitro or methyl groups, or $R_2$ represents a silyl group, for example a trialkylsilyl group.

11. Process acording to claim 10 wherein $R_2$ is methyl, t-butyl, 2-bromoethyl, 2,2,2-trichloroethyl, benzyl, 4-nitrobenzyl, 4-methoxybenzyl, benzhydryl or trimethylsilyl.

12. Process according to claim 1, wherein a compound of formula III is separated from the reaction mixture, optionally purified and treated with a debrominating agent in the presence of a hydrogen donor.

## Revendications

1. Procédé perfectionné de préparation de dérivés du 3-bromométhyl-3-céphème-sulfoxyde de formule générale:

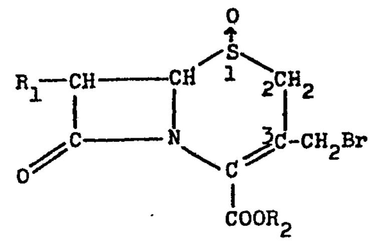

I

où $R^1$ représente un radical acylamido et $R^2$ représente un radical protecteur du radical carboxyle, par bromation de manière connue en soi d'un désacétoxy-3-céphème-sulfoxyde de formule générale:

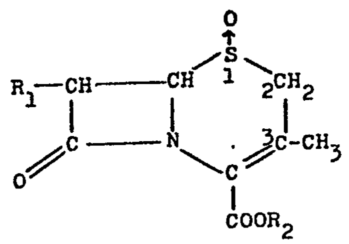

II

ou $R^1$ et $R^2$ sont tels que défini ci-dessus, pour la conversion, entre autres, du radical méthyle en la position 3 d'un tel composé en un radical bromométhyle, caractérisé en ce qu'on traite le mélange de réaction qui est obtenu après que la bromation a été effectuée et qui contient un composé de formule générale:

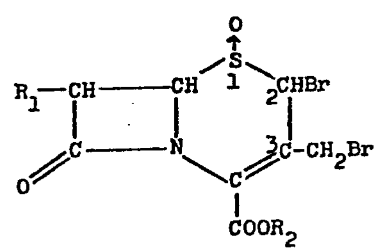

III

ou $R^1$ et $R^2$ sont tels que défini ci-dessus, ou éventuellement un composé de formule générale III séparé d'un tel mélange de réaction, à l'aide d'un agent de débromation choisi dans la classe consistant en les composés organiques du phosphore trivalent et les sels inorganiques qui sont capables de remplacer, en présence d'un conneur d'hydrogène, l'atome de brome en la position 2 par un atome d'hydrogène, et on isole suivant des techniques connues en soi le dérivé de 3-bromométhyl-3-céphème-sulfoxyde de formule générale I ainsi produit.

2. Procédé suivant la revendication 1, suivant lequel le composé organique du phosphore trivalent est choisi dans la classe consistant en

(a) les phosphites de formule générale:

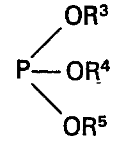

IV

ou $R^3$, $R^4$ et $R^5$, identiques ou différents, représentent chacun un radical alkyle en chaîne droite ou ramifiée qui peut être substitué par un atome de chlore et comprend 1 à 18 atomes de carbone, un radical phényle (qui peut être substitué par un radical méthyle ou un atome de chlore), un radical allyle ou un radical benzyle;

(b) d'autres phosphites, par exemple le phosphite de phényle et de népentylglycol, la phosphite de polydipropylèneglycol et de phényle, le phosphite de tris 25 et le phosphite de tris(dipropylèneglycol);

(c) des di- et triphosphites, par exemple le diphosphite de diisodécyle et de pentaérythritol, le diphosphite de tétraphényle et de dipropylèneglycol, le diphosphite de distéaryle et de pentaérythritol, le diphosphite de diphényle de didécyle et de 2,2,4-triméthyl-1,3-pentanediol, le diphosphite de tétrakis(nonylphényle) et de polypropylèneglycol et le triphosphite d'heptakis(dipropylèneglycol);

(d) des phosphonites, par exemple des phénylphosphonites de di(alkyle inférieur);

(e) des phosphinites, par exemple des diphénylphosphinites d'alkyle inférieur;

(f) des phosphines, par exemple la chlorodiphénylphosphine;

(g) d'autres composés du phosphore trivalent, par exemple le triamide hexaméthylphosphoreux, le 2-éthoxy-4-méthyl-1,3,2-dioxaphospholane, le 4-méthyl-2-piperidino-1,3,2-dioxaphospholane, le N-méthyl-N-phénylphosphoramidite de diéthyle et le pyrophosphite de bis-o-phénylène.

3. Procédé suivant la revendication 1, suivant lequel le sel inorganique est le chlorure stanneux, le sulfite de sodium ou le dithionite de sodium.

4. Procédé suivant la revendication 1 ou 2, suivant lequel la quantité d'agent de débromation est en excès de la quantité du composé de formule III.

5. Procédé suivant la revendication 1 ou 3, suivant lequel l'agent de débromation est utilisé en quantité équimolaire par rapport au composé de formule III.

6. Procédé suivant la revendication 2, suivant lequel le composé organique du phosphore trivalent est le phosphite de triméthyle, le phosphite de triéthyle, le phosphite de tripropyle, le phosphite de triisopropyle, le phosphite de tributyle, le phosphite de triisobutyle, le phosphite de triamyle, le phosphite de trioctyle, le phosphite de triisooctyle, le phosphite de tris(2-éthylhexyle), le phosphite de tridécyle, le phosphite de triisodécyle, le phosphite de trinéodécyle, le phosphite de trilauryle, le phosphite de tristéaryle, le phosphite de triallyle, le phosphite de tris(2-chloroéthyle), le phosphite de triphényle, le phosphite de tris(p-méthylphényle), le phosphite de tris-o-tolyle, le phosphite de tris(p-chlorophényle), le phosphite de 2-éthylhexyle et de dioctyle, le phosphite de diéthyle et de benzyle, le phosphite de didécyle et de phényle, le phosphite de diisooctyle et de phényle, le phosphite d'isooctyle et de diphényle, le phosphite de 2-éthylhexyle et de diphényle, le phosphite de décyle et de diphényle, le phosphite d'isodécyle et de diphényle, le phénylphosphonite de diméthyle, le phénylphosphonite de diéthyle, le phénylphosphonite de di-t-butyle, le diphenylphosphinite de méthyle ou le diphénylphosphinite d'éthyle.

7. Procédé suivant la revendication 2, suivant lequel le composé organique du phosphore trivalent est le phosphite de triméthyle, le phosphite de triéthyle, le phosphite de tributyle, le phosphite de triisooctyle, le phosphite de triisodécyle, le phosphite de trilauryle, le phosphite de tristéaryle, le phosphite de triallyle, le phosphite de tris(2-chloroéthyle), le phosphite de triphényle, le phosphite de tris-o-tolyle, le phosphite de tris(p-chlorophényle), le phosphite de 2-éthylhexyle et de dioctyle, le phosphite de diéthyle et de benzyle, le phosphite d'isooctyle et de diphényle, le phosphite de 2-éthylhexyle et de diphényle, le phosphite d'isodécyle et de diphényle, le phosphite de phényle et de néopentylglycol, le phosphite de phényle et de poly(dipropylglycol), le phosphite de tris 25, le phosphite de tris(dipropylèneglycol), le diphosphite de diisodécyle et de pentaérythritol, le diphosphite de tétraphényle et de dipropylèneglycol, le diphosphite de distéaryle et de pentaérythritol, le diphosphite de diphényle, de didécyle et de 2,2,4-triméthyl-1,3-pentanediol, le diphosphite de tétrakis (nonylphényle) et de polypropylèneglycol, le triphosphite d'heptakis(dipropylèneglycol), le pyrophosphite de bis-o-phényléne, le triamide hexaméthylphosphoreux, le 2-éthoxy-4-méthyl-1,3,2-dioxaphospholane, le 4-méthyl-2-pipéridino-1,3,2-dioxaphospholane, le N-méthyl-N-phénylphosphoramidite de diéthyle, le phénylphosphonite de diéthyle, le diphénylphosphonite de méthyle ou la chlorodiphénylphosphine.

8. Procédé suivant la revendication 1, suivant lequel le donneur d'hydrogène est l'eau, un alcanol inférieur, par exemple le méthanol, ou un acide carboxylique, par exemple l'acide acétique.

9. Procédé suivant la revendication 1, suivant lequel $R^1$ représente un radical phénylacétamido, phénoxyacétamido, benzamido ou formamido.

10. Procédé suivant la revendication 1, suivant lequel $R^2$ représente un radical alkyle en chaîne droite ou ramifiée comptant 1 à 4 atomes de carbone éventuellement substitué par un ou plusieurs atomes d'halogène ou par un ou deux radicaux phényle, lesquels radicaux phényle peuvent eux-mêmes porter un ou plusieurs substituants, par exemple des radicaux nitro ou méthoxy, ou bien $R^2$ représente un radical silyle, par exemple un radical trialkylsilyle.

11. Procédé suivant la revendication 10 suivant lequel $R^2$ représente un radical méthyle, t-butyle, 2-bromoéthyle, 2,2,2-trichloroéthyle, benzyle, 4-nitrobenzyle, 4-méthoxybenzyle, benzhydryle ou triméthylsilyle.

12. Procédé suivant la revendication 1, suivant lequel on sépare un composé de formule III du mélange de réaction, on le purifie éventuellement et on le traite à l'aide d'un agent de débromation en présence d'un donneur d'hydrogène.

**Patentansprüche**

1. Verbessertes Verfahren zur Herstellung von 3-Brommethyl-3-cephem-sulfoxidderivaten der allgemeinen Formel I

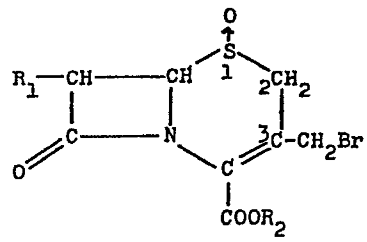

I

worin $R_1$ eine Acylamidogruppe und $R_2$ eine den Carboxylrest schützende Gruppe bedeutet, durch Bromieren eines Desacetoxy-3-cephem-sulfoxids der allgemeinen Formel II

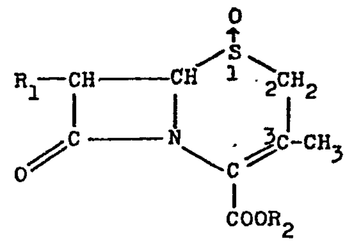

II

worin $R_1$ und $R_2$ die obige Bedeutung haben, nach bekannten Verfahren, um unter anderem die Methylgruppe in 3-Stellung einer solchen Verbindung in eine Brommethylgruppe überzuführen, dadurch gekennzeichnet, dass man das nach dem Bromieren erhaltene Reaktionsgemisch, welches eine Verbindung der allgemeinen Formel III

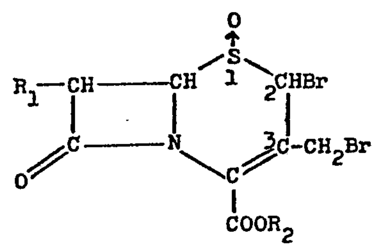

III

worin $R_1$ und $R_2$ die obige Bedeutung haben, enthält, oder gegebenenfalls die vom Reaktionsgemisch abgetrennte Verbindung der allgemeinen Formel III mit einem Endbromierungsmittel aus der Gruppe von dreiwertigen organischen Phosphorverbindungen und anorganischen Salzen, welche in Gegenwart eines Wasserstoffdonators das Bromatom in 2-Stellung durch Wasserstoff ersetzen können, behandelt, und dass derart erhaltene 3-Brommethyl-3-cephem-sulfoxidderivat der allgemeinen Formel I nach bekannten Verfahren gewinnt.

2. Verfahren nach Anspruch 1, dadurch gekennzeichnet, dass die dreiwertige organische Phosphorverbindung aus der nachfolgenden Gruppe gewählt wird:

(a) Phosphite der allgemeinen Formel IV

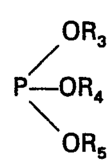

IV

worin $R_3$, $R_4$ und $R_5$ gleich oder verschieden sind und jedes für eine geradkettige oder verzweigte Alkylgruppe steht, die durch ein Chloratom substituiert sein kann und 1 bis 18 Kohlenstoffatome aufweist, eine gegebenenfalls durch eine Methylgruppe oder Chloratom substituierte Phenylgruppe, eine Allyl- oder Benzylgruppe steht;

(b) andere Phosphite, beispielsweise Phenylneopentylglycolphosphit, Poly(dipropylenglycol)-phenylphosphit, Tris-25-phosphit und Tris(dipropylenglycol)phosphit;

(c) Di- und Triphosphite, beispielsweise Diisodecylpentaerythritol-diphosphit, Tetraphenyldipropylenglycoldiphosphit, Distearyl-pentaerythritol-diphosphit, Diphenyl-didecyl-(2,2,4-trimethyl-1,3-pentandiol)-diphosphit, Tetrakis(nonylphenyl)polypropylenglycol-diphosphit und Heptakis(dipropylenglycol)triphosphit;

(d) Phosphonite, beispielsweise Di(niederalkyl)phenylphosphonite;

(e) Phosphinite, beispielsweise Niederalkyl-diphenylphosphinite;

(f) Phosphine, beispielsweise Chlordiphenylphosphin;

(g) andere dreiwertige Phosphorverbindungen, beispielsweise Hexamethylphosphortriamid, 2-Aethoxy-4-methyl-1,3,2-dioxaphospholan, 4-Methyl-2-piperidino-1,3,2-dioxaphospholan, Diäthyl-N-methyl-N-phenylphosphoramidit und Bis-o-phenylen-pyrophosphit.

3. Verfahren nach Anspruch 1, dadurch gekennzeichnet, dass das anorganische Salz Zinn-IIchlorid, Natriumsulfit oder Natriumdithionit ist.

4. Verfahren nach Anspruch 1 oder 2, dadurch gekennzeichnet, dass ein Ueberschuss an Endbromierungsmittel, bezogen auf die Menge der Verbindung der Formel III, eingesetzt wird.

5. Verfahren nach Anspruch 1 oder 3, dadurch gekennzeichnet, dass das Endbromierungsmittel in äquimolaren Mengen, bezogen auf die Verbindung der Formel III, eingesetzt wird.

6. Verfahren nach Anspruch 2, dadurch gekennzeichnet, dass die dreiwertige organische Phosphorverbindung Trimethylphosphit,Triäthylphosphit, Tripropylphosphit, Triisopropylphosphit, Tributylphosphit, Triisobutylphosphit, Triamylphosphit, Trioctylphosphit, Triisooctylphosphit, Tris(2-äthylhexyl)phosphit, Tridecylphosphit, Triisodecylphosphit, Trineodecylphosphit, Trilaurylphosphit, Tristearylphosphit, Triallylphosphit, Tris(2-chloräthyl)phosphit, Triphenylphosphit, Tris(p-methylphenyl)phosphit, Tris-o-tolylphosphit, Tris(p-chlorphenyl)phosphit, 2-Aethylhexyldioctylphosphit, Diäthylbenzylphosphit, Didecylphenylphosphit, Diisodecylphenylphosphit, Isooctyldiphenylphosphit, 2-Aethylhexyldiphenylphosphit, Decyldiphenylphosphit, Isodecyldiphenylphosphit, Dimethylphenylphosphonit, Diäthylphenylphosphonit, Di(t-butyl)phenylphosphonit, Methyldiphenylphosphonit oder Aethyldiphenylphosphinit ist.

7. Verfahren nach Anspruch 2, dadurch gekennzeichnet, dass die dreiwertige organische Phosphorverbindung Trimethylphosphit, Triäthylphosphit, Tributylphosphit, Triisooctylphosphit, Triisodecylphosphit, Trilaurylphosphit, Tristearylphosphit, Triallylphosphit, Tris(2-chloräthyl)phosphit, Triphenylphosphit, Tris-o-tolyl-phosphit, Tris(p-chlorphenyl)phosphit, 2-Aethylhexyl-dioctylphosphit, Diäthylbenzylphosphit, Isooctyldiphenylphosphit, 2-Aethylhexyldiphenylphosphit, Isodecyldiphenylphosphit, Phenylneopentylglycolphosphit, Poly(dipropylenglycol)phenylphosphit, Tris-25-phosphit, Tris(dipropylenglycol)phosphit, Diisodecylpentaerythritoldiphosphit, Tetraphenyldipropylenglycoldiphosphit, Diestearylpentaerythritoldiphosphit, Diphenyldidecyl(2,2,4-trimethyl-1,3-pentandiol)diphosphit, Tetrakis(nonylphenyl)polypropylenglycoldiphosphit, Heptakis(dipropylenglycol)triphosphit, Bis-o-phenylenpyrophosphit, Hexamethylphosphortriamid, 2-Aethoxy-4-methyl-1,3,2-dioxaphospholan, 4-Methyl-2-piperidino-1,3,2-dioxaphospholan, Diäthyl-N-methyl-N-phenylphosphoramidit, Diäthylphenylphosphonit, Methyldiphenylphosphinit oder Chlordiphenylphosphin ist.

8. Verfahren nach Anspruch 1, dadurch gekennzeichnet, dass der Wasserstoffdonator Wasser, ein niederes Alkanol, beispielsweise Methanol oder eine Carbonsäure, beispielsweise Essigsäure, ist.

9. Verfahren nach Anspruch 1, dadurch gekennzeichnet, dass $R_1$ Phenylacetamido, Phenoxyacetamido, Benzamido oder Formamido bedeutet.

10. Verfahren nach Anspruch 1, dadurch gekennzeichnet, dass $R_2$ eine geradkettige oder verzweigte Alkylgruppe mit 1 bis 4 Kohlenstoffatomen, die gegebenenfalls durch ein oder mehrere Halogenatome oder eine oder mehrere Phenylgruppen substituiert sein kann, bedeutet, wobei die Phenylgruppen eine oder mehrere Substituenten, beispielsweise Nitro- oder Methoxygruppen, aufweisen können, oder $R_2$ für eine Silylgruppe, beispielsweise Trialkylsilylgruppe steht.

11. Verfahren nach Anspruch 10, dadurch gekennzeichnet, dass $R_2$ für Methyl, t-Butyl, 2-Bromäthyl, 2,2,2-Trichloräthyl, Benzyl, 4-Nitrobenzyl, 4-Methoxybenzyl, Benzhydryl oder Trimethylsilyl steht.

12. Verfahren nach Anspruch 1, dadurch gekennzeichnet, dass eine Verbindung der Formel III aus dem Reaktionsgemisch abgetrennt, gegebenenfalls gereinigt und mit einem Endbromierungsmittel in Gegenwart eines Wasserstoffdonators behandelt wird.